# EUROPÄISCHE PATENTANMELDUNG

(11) **EP 3 575 335 A1**
(43) Veröffentlichungstag der Anmeldung: **04.12.2019**
(21) Anmeldenummer: 18175050.6
(22) Anmeldetag: 30.05.2018
(51) Int. Cl.: C08G 18/32, C08G 65/26

(54) **VERFAHREN ZUR HERSTELLUNG EINES ALKOXYLIERTEN PRODUKTES**

(71) Anmelder: Hexion GmbH, 58642 Iserlohn-Letmathe (DE)
(72) Erfinder: Schröter, Stephan, 45259 Essen (DE); Kukkala, Pravin, Louisville, KY, 40210 (US); Viswanathan, Ganapathy, Louisville, KY 40223 (US); Kerkaidou, Athina, 58644 Iserlohn (DE); Bay, Sarah, 40227 Düsseldorf (DE)

(57) **Zusammenfassung**

Die Erfindung betrifft ein Verfahren zur Herstellung eines alkoxylierten Produktes.

Um aromatische Polyole zur Herstellung von Polymeren auf Polyurethan- und Polyisocyanuratbasis zur Verfügung zu stellen, die eine gute Vermischbarkeit mit der Isocyanatkomponente und weitereren Komponenten gewährleisten, eine gute Lagerbeständigkeit aufweisen und dem Endprodukt eine gute Flammfestigkeit verleihen, wird ein Verfahren zur Herstellung eines alkoxylierten Produktes durch Umsetzung von Bisphenol F mit Propylencarbonat und/oder Propylenoxid als Propoxylierungsmittel und Ethylencarbonat und/oder Ethylenoxid als Ethoxylierungsmittel vorgeschlagen, wobei das Propoxylierungsmittel und das Ethoxylierungsmittel in einem molaren Verhältnis von 70:30 bis 45:55 verwendet wird.

## Beschreibung

Bei der Herstellung von Polyurethanen und Polyisocyanuraten werden Isocyanate unter Verwendung von Polyolen durch eine Polyadditionsreaktion vernetzt. Die Isocyanate weisen mindestens zwei -NCO-Gruppen und die Polyole mindestens zwei reaktionsfähige -OH-Gruppen (mehrwertige Alkohole) auf. Es entstehen Polymere die je nach ihrem chemischen oder morphologischen Aufbau thermoplastische, elastische oder duroplastische Eigenschaften aufweisen können. Demzufolge besitzen Polyurethane und Polyisocyanurate ein sehr breites Anwendungsgebiet, wie z.B. für Schäume (hart oder flexibel), Beschichtungen, Klebstoffe, Elastomere, Isolationen und Kompositwerkstoffe. Insbesondere bei der Suche nach energie- und ressourceneffizienten Werkstoffen kommen den Polyurethanen und Polyisocyanuraten aufgrund einfach zu realisierenden Leichtbaukonstruktionen eine besonders große Bedeutung zu.

Die Herstellung von Polyurethanen und Polyisocyanuraten erfolgt in der Art, dass Polyole mit Isocyanten gemischt werden, wobei nach kurzer Zeit das System zu gelieren beginnt. Es ist ersichtlich, dass die Komponenten hinsichtlich ihrer Viskositäten aufeinander abgestimmt sein müssen, damit ein Vermischungsgrad vorliegt, der zu homogenen Produkten mit gewünschten Eigenschaften führt. Eigenschaften des Endproduktes werden im Wesentlichen durch die Kettenlänge und Verzweigungsgrad der Polyolkomponente bestimmt, so dass häufig auch Kombinationen aus verschiedenen Polyolen eingesetzt werden, z.B. Polyetherpolyole und Polyesterpolyole, um Verarbeitung und Eigenschaften zu optimieren.

Bei der Herstellung typischer Polyurethan-Hartschaumstoffe führt die stark exotherme Reaktion zwischen Polyolen und Isocyanaten zu innerer Verkrustung. Dieses Phänomen beeinträchtigt die physikalischen Eigenschaften des Schaums nachteilig und erhöht das Potential, Probleme hinsichtlich der Entflammbarkeit zu verursachen. Darüber hinaus erhöht die Verwendung von organischen Treibmitteln auf Kohlenwasserstoffbasis die Entflammbarkeit der fertigen Schaumstoffe. Folglich werden flammhemmende Additive in die Formulierung gegeben, wobei dies im Allgemeinen halogenierte Verbindungen sind. Viele solcher Flammschutzmittel stellen jedoch eine Gefahr für die Umwelt dar. Daher ist es wünschenswert, Polyole zu verwenden, die von Natur aus schwer entflammbar sind, die die Wärme- und Feuerbeständigkeit der Polyurethan (PUR) - und Polyisocyanurat (PIR) - Schäume verbessern und möglicherweise die Mengen dieser umweltfreundlichen und teuren Flammschutzadditive in der Formulierung minimieren.

Alkoxylierte Bisphenole sind für die Herstellung von Polyurethanen bekannt. So beschreibt die EP 0 763 067 B1 die Verwendung von alkoxylierten Bisphenolen für die Herstellung von Schmelzklebern und die EP 2 743 285 A1 für beschichtete Leitungselemente. Weiterhin kann der EP 1 851 261 B1 entnommen werden, dass eine Komponente einer zweikomponentigen Polyurethanzusammensetzungen für Strukturklebstoffe ein ethoxyliertes oder propoxyliertes aromatisches Diol in Kombination mit aliphatischen Triolen sein kann.

Weiterhin hat sich herausgestellt, dass aromatische Polyole vom Dioltyp auf der Basis von Bisphenol A für die Herstellung von Polyurethanen nur unzureichend geeignet sind, da sie feste Substanzen sind, was verarbeitungstechnisch sehr nachteilig ist und außerdem noch eine schlechte thermische Beständigkeit besitzen.

Es hat sich auch gezeigt, dass die Verwendung von sowohl reinem ethoxylierten Bisphenol F als auch reinem propoxylierten Bisphenol F als aromatische Polyetherpolyole zur Herstellung von Polyurethanen eine ernste Herausforderung darstellt. Grund hierfür ist, dass es sich jeweils um pastöse Substanzen handelt, die bei 20 bis 30 °C nicht pumpfähig sind und somit nicht die gewünschte prozesstechnische Verarbeitungsfähigkeit in der Polyurethanherstellung besitzen. Ein Aufschmelzen des ethoxylierten Bisphenol F bzw. des propoxylierten Bisphenol F setzt ein Energieeintrag in das System voraus, was auch nicht erwünscht ist. Weiterhin führten Versuche reines ethoxyliertes Bisphenol F als auch reines propoxyliertes Bisphenol F in Lösung zu bringen dazu, dass sie kaum in geeigneten Lösungsmittel aufgelöst werden konnten, da sie leicht zur Kristallisation neigten.

Es ist daher Aufgabe der hier vorliegenden Erfindung aromatische Polyole zur Herstellung von Polymeren auf Polyurethan- und Polyisocyanuratbasis zur Verfügung zu stellen, die eine gute Vermischbarkeit mit der Isocyanatkomponente und weiteren Komponenten gewährleisten, eine gute Lagerbeständigkeit aufweisen und dem Endprodukt eine gute Flammfestigkeit verleihen, wobei auf den Einsatz von halogenierten Verbindungen verzichtet werden kann.

Gelöst wird diese Aufgabe erfindungsgemäß durch ein Verfahren zur Herstellung eines alkoxylierten Produktes durch Umsetzung von Bisphenol F mit Propylencarbonat und/oder Propylenoxid als Propoxylierungsmittel und Ethylencarbonat und/oder Ethylenoxid als Ethoxylierungsmittel, wobei das Propoxylierungsmittel und das Ethoxylierungsmittel in einem molaren Verhältnis von 70:30 bis 45:55 verwendet wird.

Alkoxyliertes Bisphenol F, was durch Umsetzung eines Ethoxylierungsmittel und eines Propoxylierungsmittel mit Bisphenol F entstanden ist, stellt anmeldungsgemäß ein Co-Kondensat dar.

Bevorzugt werden auf ein Mol Bisphenol F 1,6 mol bis 0,9 mol Propoxylierungsmittel und 1,6 mol bis 0,9 mol Ethoxylierungsmittel, wiederum bevorzugt 1,3 mol bis 1,0 mol Propoxylierungsmittel und 1,3 mol bis 1,0 mol Ethoxylierungsmittel verwendet. Es hat sich dabei gezeigt, dass ein molares Verhältnis Bisphenol F: Summe aus Propoxylierungsmittel und Ethoxylierungsmittel von z.B. 1:2,0 bis 1:3,2 einen positiven Einfluss auf die Lagerstabilität des reinen Co-Kondensates, wie auch in Lösung hat.

Eine besonders überraschende Wirkung hinsichtlich der Lagerstabilität in reiner Form als auch in Lösungsmitteln wurde bei einem molaren Verhältnis von Bisphenol F : Propoxylierungsmittel: Ethoxylierungsmittel von 1:1:1 erreicht.

Das zur Herstellung von alkoxyliertem Bisphenol F verwendete Bisphenol F ist aus dem Stand der Technik bekannt. So wird es durch Umsetzung von Phenol mit Formaldehyd im Sauren hergestellt. Es entsteht ein Isomerengemisch aus o-o', o-p und p-p' Bisphenol F, was je nach Reaktionsbedingungen und Herstellverfahren in der Zusammensetzung variieren kann. Die Hydroxyfunktionalität des hergestellten Bisphenol F beträgt mindestens 2.

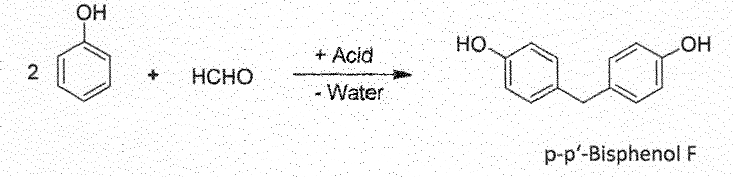

Durch Umsetzung des Bisphenol F mit Propoxylierungsmitteln, wie Propylenoxid und/oder Propylencarbonat, und mit Ethoxylierungsmitteln, wie Ethylenoxid und/oder Ethylencarbonat, in einem molaren Verhältnis von 70:30 bis 45:55, bevorzugt 50:50, entsteht das alkoxylierte Bisphenol F. Da die Umsetzung mit Ethylenoxid bzw. Propylenoxid, die im gasförmigen Zustand vorliegen, in einem Druckreaktor erfolgen muss, was prozesstechnisch aufwendiger ist, wird bevorzugt als Ethoxylierungsmittel Ethylencarbonat bzw. als Propoxylierungsmittel Propylencarbonat verwendet.

Am Beispiel des p-p' Bisphenol F soll die Reaktion verdeutlicht werden:

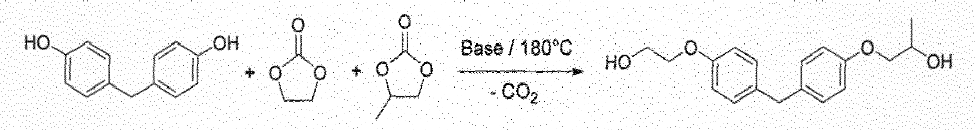

Aufgrund der verschiedenen Isomere des Bisphenol F und deren Difunktionalität entstehen auch entsprechende Isomere des alkoxylierten Bisphenol F. Insbesondere durch die Verwendung von Propylenoxid und/oder Propylencarbonat entstehen weitere Isomere, da zwei verschiedene Kohlenstoffatome zur Reaktion befähigt sind. Darüber hinaus können Sterioisomere gebildet werden.

Generell wird die Alkoxylierungsreaktion im alkalischen Medium bei Temperaturen zwischen 120 °C und 200 °C durchgeführt. In der Regel wird das Bisphenol F vorgelegt, aufgeschmolzen und es erfolgt die Zugabe eines alkalischen Mediums unter Rühren in Form von z.B. Kaliumcarbonat, Natriumcarbonat, Kaliumhydroxid, Natriumhydroxid, Kaliumalkoholate, Natriumalkoholate, Calciumhydroxid, Calciumoxid, Aminen oder Triphenylphosphin bei Temperaturen bis z. B. ca. 200 °C. Anschließend wird das Propoxylierungsmittel und das Ethoxylierungsmittel zugegeben und je nach technischer Möglichkeit das entstehende Kohlendioxid abgeführt. Für die Eigenschaften des alkoxylierten Produktes ist es unerheblich, ob das Propoxylierungsmittel zeitgleich oder zeitlich versetzt oder im Gemisch mit dem Ethoxylierungsmittel zugegeben wird. Allerdings konnte die Reaktionszeit der Alkoxylierung verkürzt werden, wenn das Propoxylierungsmittel, insbeondere Propylencarbonat, zuerst mit dem Bisphenol F zur Reaktion gebracht wurde.

Das entstandene alkoxylierte Produkt wird gegebenenfalls zur Verringerung des Wassergehaltes bzw. freien Propoxylierungsmittel- und/oder Ethoxylierungsmittelgehaltes unter Vakuum destilliert und kann nach Abkühlung gegebenenfalls mit einer Säure neutralisiert werden. Ein neutrales Produkt wird bevorzugt bei der Anwendung als Polyol bei der Polyurethanherstellung. Bevorzugt ist eine Neutralisation z.B. mit einer kompatiblen organischen (wie z.B. Benzoesäure, Phthalsäure, Milchsäure, Anthranilsäure oder Salicylsäure und/oder anorganischen Säure (wie z.B. Salzsäure, Schwefelsäure, Phosphorsäure oder Salpetersäure).

Generell ist es auch möglich die Herstellung des Bisphenol F durch Reaktion von Phenol mit Formaldehyd in einem Reaktor durchzuführen und direkt anschließend vorzugsweise in demselben Reaktionsgefäß die Umsetzung mit dem Propoxylierungsmittel und dem Ethoxylierungsmittel vorzunehmen. Das hat den Vorteil, dass eine Lagerung des Bisphenol F nicht erforderlich wäre und bedarfsgerecht sofort weiterverwendet werden kann.

Das erfindungsgemäß hergestellte alkoxylierte Produkt zeigt überraschenderweise eine hervorragende Löslichkeit in Lösungsmitteln, die insbesondere mit der Herstellung von Polyurethanen kompatibel sind. So löst sich das alkoxylierte Produkt hervorragend in z.B.

Organophosphaten wie Triethylphosphat und Diphenylkresylphosphat, 1,4-Butandiol, alkoxyliertem Resorcin wie propoxyliertem Resorcin, Polyetherpolyole wie ethoxylierter Zucker, aromatische Polyesterpolyole, modifizierten oder unmodifizierten phenolischen Resolen (z.B. Phenol und Kresol basierte Resole) allein oder in Mischungen hiervon. Die Resole können auch in organischem Lösungsmittel gelöst vorliegen.

Die Kristallisationsneigung des erfindungsgemäß hergestellten alkoxylierten Produktes ist überraschenderweise im Vergleich zu reinem ethoxylierten Bisphenol F oder propoxylierten Bisphenol F enthaltenden Lösungen sehr gering bzw. nicht vorhanden. Besonders überraschend war, dass die Umsetzung von Bisphenol F mit Propylencarbonat und/oder Propylenoxid als Propoxylierungsmittel und Ethylencarbonat und/oder Ethylenoxid als Ethoxylierungsmittel, wobei das Propoxylierungsmittel und das Ethoxylierungsmittel in einem molaren Verhältnis von 70:30 bis 45:55 vorliegen, die Lagerstabilität sowohl des alkoxylierten Produktes wie auch in Lösung gebracht um ein Vielfaches übersteigt als außerhalb des beanspruchten molaren Verhältnisses. Außerdem verleiht das erfindungsgemäß hergestellte Produkt durch seinen hohen aromatischen Anteil dem Endprodukt eine höhere Flammenbeständigkeit und Kompatibilität zu Diisocyanaten (z.B. MDI) oder Polyisocyanaten (z.B. p-MDI) sowie zu Treibmitteln.

Das alkoxylierte Produkt wurde bevorzugt im Gewichtsverhältnis zum Lösungsmittel 90:10 bis 10:90, bevorzugt 60:40 bis 40:60, wiederum bevorzugt 50:50 zugegeben. Insbesondere bei einem nach dem erfindungsgemäßen Verfahren hergestellten alkoxylierten Produkt: Lösungsmittel-Verhältnis von 50:50 konnte eine über mehrere Wochen sehr gute Lagerstabilität in verschiedenen Lösungsmitteln erzielt werden.

Als bevorzugt hat sich als Lösungsmittel alkoxyliertes Resorcin bewährt. Unter dem Begriff "alkoxyliertes Resorcin" fallen Substanzen, bei denen das Resorcin mit mindestens einem Alkoxylierungsmittel, so z.B. einem Ethoxylierungsmittel (Ethylenoxid, Ethylencarbonat) und/oder Propoxylierunsgmittel (Propylenoxid, Propylencarbonat) umgesetzt wurde. Das molare Verhältnis von Resorcin (Resorcinol) zu Alkoxylierunsgmittel beträgt bevorzugt 1: 2 bis 1: 2,5.

Unter alkoxyliertem Resorcin ist z.B. folgende Struktur zu fassen: 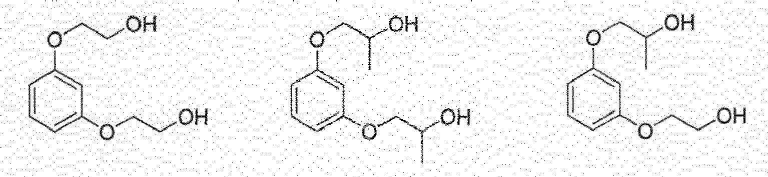 wobei diese Produkte hauptsächlich bei der Reaktion von Resorcin mit Ethylencarbonat und Propylencarbonat entstehen. Es können aber noch weitere Produkte, insbesondere bei der Reaktion mit Propylencarbonat gebildet werden, da in Propylencarbonat zwei unterschiedliche C-Atome angegriffen werden. Ebenso werden verschiedene Stereoisomere bei der Reaktion mit Propylencarbonat erhalten.

Besonders bevorzugt ist, wenn als Alkoxylierungsmittel eine Kombination aus einem Ethoxylierungsmittel und einem Propoxylierungsmittel verwendet wird. Ein solch alkoxyliertes Resorcin wirkte als Lösungsmittel für das erfindungsgemäß alkoxylierte Produkt etwas wirksamer als z.B. reines propoxyliertes Resorcin.

Die Herstellung des alkoxylierten Resorcins kann in der Art erfolgen, dass das Resorcin aufgeschmolzen wird und im alkalischen Medium in Form von z.B. Kaliumcarbonat, Natriumcarbonat, Kaliumhydroxid, Natriumhydroxid, Kaliumalkoholaten, Natriumalkoholaten, Calciumhydroxid, Calciumoxid, Aminen oder Triphenylphosphin bei erhöhten Temperaturen mit einem ersten Alkoxylierungsmittel (z.B. Propylencarbonat) versetzt wird und die Reaktion unter Abführung von Kohlendioxid stattfindet. Daran anschließen kann sich gegebenenfalls die Zugabe eines weiteren Alkoxylierungsmittel (z.B. Ethylencarbonat) bei erhöhter Temperatur, wobei das Kohlendioxid wiederum abgeleitet wird. Nach entsprechender Nachreaktion kann das Produkt gegebenenfalls abdestilliert und mit einer Säure (wie z.B. Benzoesäure, Phthalsäure, Milchsäure, Anthranilsäure, Salicylsäure, Salzsäure, Schwefelsäure, Phosphorsäure und/oder Salpetersäure) neutralisiert werden.

Die Verwendung von alkoxylierten Resorcin als Lösungsmittel ist insbesondere bei der Herstellung von Polyurethanen von Vorteil, da zusätzliche difunktionelle Gruppen vorhanden sind, die mit Diisocyanaten reagieren können. Gleichzeitig wird die Viskosität der gesamten Polyolmischung reduziert. Weiterhin erhöht die Verwendung von alkoxylierten Resorcin den aromatischen Anteil der Polyolkomponente, so dass die Flammbeständigkeit und die Kompatibilität z.B. zum MDI oder den Treibmitteln weiter erhöht werden konnte.

Verfahrenstechnisch günstig ist es, wenn bei der Herstellung des erfindungsgemäß alkoxylierten Produktes das Resorcin gleichzeitig oder im Anschluss unter Ausnutzung desselben Reaktionsgefäßes alkoxyliert wird. Damit kann z. B. eine Polyolkomponente einfach bereitgestellt werden, die aufgrund ihrer Funktionalität und Viskosität zur Reaktion mit Polyisocyanaten geeignet ist. Aufgrund der hervorragenden Lagerstabilität kann diese Polyolkomponente bedarfsgerecht zur Verfügung gestellt werden.

Generell ist es auch möglich, dass das erfindungsgemäß hergestellte alkoxylierte Produkt allein oder in Kombination mit einem Lösungsmittel als eine Polyolkomponente verwendet wird und noch weitere Polyolkomponenten zur Herstellung von Polyurethanen bzw. Polyisocyanuraten eingesetzt werden, wie z.B. Polyesterpolyole. Polyesterpolyole umfassen Reaktionsprodukte von Polyolen, üblicherweise Diolen, mit Polycarbonsäuren oder ihren Anhydriden, üblicherweise Dicarbonsäuren oder Dicarbonsäureanhydriden. Die Polycarbonsäuren oder -anhydride können aliphatisch, cycloaliphatisch, aromatisch und / oder heterocyclisch sein. Im Vergleich zu Polyesterpolyolen besitzt das erfindungsgemäß hergestellte Produkt eine höhere thermische Stabilität.

Mannich-basische Polyole, die aus Mannichbasen synthetisiert werden, können auch als Teil der isocyanatreaktiven Verbindung verwendet werden.

Als Isocyanatkomponente kommen bevorzugt m-Phenylendiisocyanat, Toluol-2,4-diisocyanat, Toluol-2,6-diisocyanat, Hexamethylen-1,6-diisocyanat, Tetramethylen-1,4-diisocyanat Cyclohexan-1,4-diisocyanat, Hexahydrotoluoldiisocyanat, Naphthylen-1,5-diisocyanat, Methoxyphenyl-2,4-diisocyanat, Diphenylmethan-4,4'-diisocyanat, 4,4'-Biphenylendiisocyanat, 3,3'-Dimethoxy-4,4'-biphenyldiisocyanat, 3,3'-Dimethyl-4,4'-biphenyldiisocyanat, 3,3'-Dimethyldiphenylmethan-4,4'-diisocyanat, 4,4',4"-Triphenylmethan Triisocyanat, ein Polymethylenpolyphenylisocyanat, polymeres Diphenylmethandiisocyanat (PMDI), Isophorondiisocyanat, Toluol-2,4,6-triisocyanat und 4,4'-Dimethyldiphenylmethan-2,2',5,5'-tetraisocyanat. In verschiedenen Ausführungsformen ist das Polyisocyanat Diphenylmethan-4,4'-diisocyanat, Diphenylmethan-2,4-diisocyanat, Hexamethylen-1,6-diisocyanat, Isophorondiisocyanat, Toluol-2,4-diisocyanat, Toluol-2,6-diisocyanat oder Mischungen davon zum Einsatz.

Diphenylmethan-4,4'-diisocyanat, Diphenylmethan-2,4-diisocyanat und Mischungen davon werden im Allgemeinen als MDI bezeichnet. Toluol-2,4-diisocyanat, Toluol-2,6-diisocyanat und Mischungen davon werden allgemein als TDI bezeichnet.

Das erfindungsgemäß hergestellte Produkt weist insbesondere mit MDI oder p-MDI bereits bei Raumtemperatur eine gute Kompatibilität auf, währenddessen bei Einsatz von rein ethoxyliertem Bisphenol F und propoxyliertem Bisphenol F höhere Temperaturen benötigt werden.

Jedes der vorstehenden Polyisocyanate kann so modifiziert werden, dass Urethan-, Harnstoff-, Biuret-, Carbodiimid-, Allophonat-, Uretonimin-, Isocyanurat-, Amid- oder ähnliche Bindungen eingeschlossen sind. Beispiele für modifizierte Isocyanate dieser Art umfassen verschiedene Urethangruppen- und / oder Harnstoffgruppen enthaltende Präpolymere und sogenannte "Flüssig-MDI" -Produkte und dergleichen. Es ist auch möglich, dass das Polyisocyanat ein blockiertes Isocyanat ist.

In Abhängigkeit von dem speziellen Typ des hergestellten Polymers und den notwendigen Eigenschaften des Polymers kann eine große Vielzahl von zusätzlichen Materialien während der Reaktion der Polyisocyanatverbindung mit dem erfindungsgemäß hergestellten Produkt vorhanden sein. Diese Materialien umfassen, ohne darauf beschränkt zu sein, Tenside, Treibmittel, Zellöffner, Füllstoffe, Pigmente und / oder Färbemittel, Trocknungsmittel, Verstärkungsmittel, Biozide, Konservierungsmittel, Antioxidantien, Verdünnungsmittel, Flammschutzmittel und dergleichen. Wenn ein Flammschutzmittel enthalten ist, kann das Flammschutzmittel ein phosphorhaltiges Flammschutzmittel sein. Beispiele für phosphorhaltige Flammschutzmittel umfassen, sind aber nicht beschränkt auf, Triethylphosphat (TEP), Triphenylphosphat (TPP), Trischlorisopropylphosphat (TCPP), Dimethylpropanphosphat, Resorcinolbis-(diphenylphosphat) (RDP), Bisphenol-A-Diphenylphosphat (BADP) und Trikresylphosphat (TCP), Dimethylmethylphosphonat (DMMP), Diphenylkresylphosphat und Aluminiumdiethylphosphinat. Erfindungsgemäß kann aufgrund des hohen aromatischen Anteil des alkoxylierten Produktes aber im Wesentlichen auf zusätzliche Flammschutzmittel verzichtet werden.

Beispiele für Verdünnungsmittel umfassen Polyglycole wie Ethylenglycol, Glycerol oder Diethylenglycol, veretherte Polyglycole wie Monomethylether von Ethylenglycol oder Dimethylether von Ethylenglycol und zweibasische Ester von Säuren wie Diethyladipat, Dimethyladipat, Diethylsuccinat oder Dimethylsuccinat. Mischungen von irgendwelchen dieser Verdünnungsmittel können ebenfalls verwendet werden.

Die relativen Mengen von Polyisocyanat und des erfindungsgemäß hergestellten Produktes werden ausgewählt, um ein Polymer zu erzeugen. Das Verhältnis dieser Komponenten wird im Allgemeinen als der "Isocyanatindex" bezeichnet, was das 100-fache des Verhältnisses von Isocyanatgruppen zu isocyanatreaktiven Gruppen bedeutet, die durch das erfindungsgemäß hergestellte Produkt bereitgestellt werden. Der Isocyanatindex beträgt im Allgemeinen mindestens 50 und kann bis zu 1000 oder mehr betragen. Starre Polymere wie Strukturpolyurethane und Hartschaumstoffe werden typischerweise unter Verwendung eines Isocyanat-Index von 90 bis 200 hergestellt. Wenn flexible oder halbflexible Polymere hergestellt werden, beträgt der Isocyanatindex im Allgemeinen 70 bis 125. Polymere, die Isocyanuratgruppen enthalten, werden oft hergestellt Isocyanat-Indizes von mindestens 150 bis zu 600 oder mehr.

Um das Polymer zu bilden, werden die Polyisocyanatverbindung und das erfindungsgemäß hergestellte alkoxylierte Produkt gemischt und gehärtet. Der Härtungsschritt wird erreicht, indem das Reaktionsgemisch Bedingungen ausgesetzt wird, die ausreichen, um die Polyisocyanatverbindung und das alkoxylierte Produkt zur Reaktion zu bringen, um das Polymer zu bilden.

Gegebenenfalls kann das Polyisocyanat und das erfindungsgemäß hergestellte Produkt - vorzugsweise in Lösungsmitteln gelöst - in verschiedenen Ausführungsformen auch einen Katalysator enthalten. Beispiele für Katalysatoren schließen tertiäre Amine, wie Dimethylbenzylamin, 1,8-Diaza-(5,4,0) undecan-7, Pentamethyldiethylentriamin, Dimethylcyclohexylamin und Triethylendiamin ein, sind aber nicht darauf beschränkt. Kaliumsalze, wie Kaliumacetat und Kaliumoctoat, können ebenfalls als Katalysatoren verwendet werden.

Das erfindungsgemäß hergestellte Produkt kann vorzugsweise zur Herstellung von Polyurethanen und Polyisocyanuraten, insbesondere Polyurethane in Form von Präpolymeren, Schäumen (fest, flexibel), Beschichtungen, Lacken, Elastomeren, Klebstoffen, Dichtmittel und/oder Kompositwerkstoffen verwendet werden.

Anhand eines Ausführungsbeispiels soll die Erfindung näher erläutert werden:
a) Herstellung von alkoxyliertem Bisphenol F (ABF)
   Molares Verhältnis von Bisphenol F: Propylencarbonat : Ethylencarbonat = 1 : 1: 1
   1. 66,64 kg Bisphenol F werden als Feststoff in einen Reaktor gegeben und bei Temperaturen zwischen 120 °C - 160 °C aufgeschmolzen.
   2. 0,17 kg Kaliumcarbonat werden anschließend unter Rühren bei 130 °C zugegeben und die Reaktionsmischung weiter auf 175 °C - 180 °C aufgeheizt.
   3. Danach werden 33,98 kg Propylencarbonat in 2,5 h unter Rühren bei 175-180 °C zugegeben. Es wird Kohlendioxid frei. Der Zulauf kann gegebenenfalls verlängert je nach technischer Möglichkeit das Kohlendioxid abzuführen.
   4. Danach werden 29,30 kg Ethylencarbonat in 2,5 h unter Rühren bei 175-180 °C zugegeben. Es wird Kohlendioxid frei. Der Zulauf kann gegebenenfalls verlängert werden je nach technischer Möglichkeit das Kohlendioxid abzuführen
   5. Für die Nachreaktion wird die Temperatur bei 175 °C bis 180 °C für 1-4 Stunden gehalten, gegebenenfalls auch länger, bis kein Kohlendioxid mehr entsteht und die Reaktion abgeschlossen ist.
   6. Es erfolgt gegebenenfalls Bestimmung des freien Wasser-, Ethylencarbonat- und Propylencarbonatgehaltes.
   7. Um eine eventuell gewünschte Reduzierung des Wasser-, Ethylencarbonat- und Propylencarbonatgehaltes vorzunehmen wird das Reaktionsgemisch 15 min unter Vakuum destilliert.
   8. Das Reaktionsgemisch wird auf 140 °C aufgeheizt und es werden 0,3 kg Salicylsäure zugesetzt.
   9. Wenn das Produkt weiter abgekühlt ist (45 °C bis 50 °C) wird es über einen Filter abgelassen. Das Produkt ist flüssig.
b) Herstellen des alkoxylierten Resorcins (Molverhältnis Resorcin : Propylencarbonat:
   Ethylencarbonat = 1 : 1,0 : 1,0)
   1. 516,7 kg Resorcin werden als Feststoff in einen Reaktor gegeben und aufgeschmolzen (Fp: 111 °C).
   2. 1,31 kg Kaliumcarbonat werden anschließend unter Rühren bei 130 °C zugegeben und die Reaktionsmischung weiter auf 175 °C - 180 °C aufgeheizt.
   3. Danach werden 479,1 kg Propylencarbonat in 2,5 h unter Rühren bei 175-180 °C zugegeben. Es wird Kohlendioxid frei. Der Zulauf kann gegebenenfalls auf bis zu 5 h verlängert werden, je nach technischer Möglichkeit das Kohlendioxid abzuführen.
   4. Anschließend werden 413,4 kg Ethylencarbonat in 2,5 h unter Rühren bei 175-180 °C zugegeben. Es wird wiederum Kohlendioxid frei. Der Zulauf kann gegebenenfalls bis zu 5 h verlängert werden, je nach technischer Möglichkeit das Kohlendioxid abzuführen.
   5. Für die Nachreaktion wird die Temperatur bei 175 °C bis 180 °C für 2-6 Stunden gehalten, gegebenenfalls auch länger, bis kein Kohlendioxid mehr entsteht und die Reaktion abgeschlossen ist.
   6. Das Reaktionsgemisch wird im Vakuum bei 175 - 180 °C kurz destilliert.
   7. Das Reaktionsgemisch wird auf 140 °C abgekühlt und es werden 2,58 kg Salicylsäure zugesetzt.

Das unter a) hergestellte alkoxylierte Bisphenol F wurde unter Verwendung des molaren Verhältnisses von Propylencarbonat: Ethylencarbonat = 50: 50 hergestellt. Die erhaltenen Eigenschaften wurden unter VI der Tabelle 1 erfasst. Entsprechend Herstellvorschrift a) wurden die restlichen Produkte der in Tabelle 1 dargestellten, hergestellt, wobei bei Herstellung von I, IV, V, VI und VII das erfindungsgemäße Verhältnis von Propoxylierungsmittel und Ethoxylierungsmittel eingehalten wurde.

Die erfindungsgemäßen alkoxylierten Bisphenol F's wiesen eine für die Polyurethanherstellung erforderliche geringe Viskosität auf. Auch handelt es sich hierbei um im Wesentlichen neutrale Produkte, wodurch sie ebenfalls für die Verwendung zur Herstellung von Polyurethanen geeignet sind.

Überraschend war zusätzlich die Lagerstabilität des erfindungsgemäß hergestellten alkoxylierten Bisphenol F (I, IV, V, VI, VII). Hierzu wurden alle Proben im reinen Zustand bei 20 °C in einen Klimaschrank gestellt. Bei den nicht erfindungsgemäß hergestellten Proben II, III, VIII- XII trat bereits nach wenigen Tagen die Kristallisation ein, die sie für die Herstellung von Polyurethanen ungeeignet macht.

Auch die Stabilität des erfindungsgemäß hergestellten alkoxylierten Bisphenol F in Lösungsmittel ist nachgewiesener Maßen deutlich besser als bei den Proben II, III, VIII - XII.

Hierzu wurde eine Mischung aus alkoxyliertem Bisphenol F, (Probe VI - hergestellt nach a)) und Triethylphosphat (TEP) in einem Gewichtsverhältnis 80 : 20 bereitet und ebenfalls bei 20 °C in den Klimaschrank gestellt. Auch hier konnte gezeigt werden, dass nur die erfindungsgemäß hergestellten alkoxylierten Produkte die gewünschte über mehrere Wochen lagerstabile Lösung lieferten.

Ergänzend hierzu wurden in Tabelle 2 weitere Mischungen aus erfindungsgemäß alkoxylierten Bisphenol F (Probe VI - hergestellt nach a) mit verschiedenen Lösungsmitteln in den angegebenen Gewichtsverhältnissen hergestellt und bei 20 °C im Klimaschrank gelagert. Die Ergebnisse sind in den Tabelle 2 dargestellt und demonstrieren überzeugend die Lagerstabilität der Mischungen, wobei als Maß der Lagerstabilität die Auskristallisation visuell beurteilt wurde.

**Tabelle 1**

| | I (Erf) | II | III | IV (Erf) | V (Erf) | VI (Erf) | VII (Erf) | VIII | IX | X | XI | XII |
|---|---|---|---|---|---|---|---|---|---|---|---|---|
| Molares Verhältnis | | | | | | | | | | | | |
| BPF: PC : EC | 1 : 1,05 : 1,05 | 1 : 1,8 : 0,2 | 1 : 1,5 : 0,5 | 1 : 1,3 : 0,7 | 1 : 1,2 : 0,8 | 1 : 1 : 1 | 1 : 1,3 : 1,3 | 1:0,8: 1,2 | 1 : 0,7 : 1,3 | 1 : 0,5: 1,5 | 1:0,4: 1.6 | 1 : 0,2 : 1,8 |
| Verhältnis | | | | | | | | | | | | |
| PC : EC | 50:50 | 90 : 10 | 75:25 | 65 : 35 | 60 : 40 | 50 : 50 | 50 : 50 | 40 : 60 | 35 : 65 | 25 : 75 | 20 : 80 | 10 : 90 |
| Viskosität 25°C [mPas] | 55680 | 280000 | 179200 | 230400 | 140800 | 130000 | 40960 | 133120 | 128000 | 140800 | 368640 | 149758 |
| Viskosität 50°C [mPas] | 1260 | 2500 | 2200 | 2320 | 2040 | 1800 | 6720 | 1920 | 1720 | 1840 | 2040 | 1277 |
| | | | | | | | | | | | | |
| Stabilität 80 % in TEP bei 20°C | x | 1 Tag | 3 Tage | ca. 4 Wochen | 2,5 Wochen | 9 Wochen | >9 Wochen | 3 Tage | < 1 Woche | 3 Tage | 1 Tag | 2 Tage |
| Stabiliät rein bei 20°C | 2 Wochen | 1 Tag | 3 Tage | ca. 13 Wochen | 2,5 Wochen | 10 Wochen | >10 Wochen | 3 Tage | < 1 Woche | 3 Tage | 1 Tag | 2 Tage |

**Tabelle 2**

| Lösungsmittel (LM) | Gewichtsverhältnis | Lagerung bei 20 °C [in Wochen] | | | | | |
|---|---|---|---|---|---|---|---|
| | | 1 | 2 | 3 | 4 | 5 | 6 |
| | ABF : LM | Auskristallisation | | | | | |
| Triethylphosphat (TEP) | 70:30 | 0% | 0% | 0% | 0% | 0% | 0% |
| | 50:50 | 0% | 0% | 0% | 0% | 0% | 0% |
| Diethylenglykol (DEG) | 70:30 | 0% | 0% | 0% | 0% | 0% | 0% |
| | 50:50 | 0% | 0% | 0% | 0% | 0% | 0% |
| TEP / DEG | 70:30 | 0% | 0% | 0% | 0% | 0% | 0% |
| Ethoxylierter Zucker (Su-EO) | 70:30 | 0% | 0% | 0% | 0% | 0% | 0% |
| | 50:50 | 0% | 0% | 0% | 0% | 1% | 1% |
| Alkoxyliertes Resorcin | 70:30 | 0% | 0% | 0% | 0% | 0% | 0% |
| | 50:50 | 0% | 0% | 0% | 0% | 0% | 0% |
| 1,4-Butandiol | 70:30 | 0% | 0% | 0% | 0% | 0% | 0% |
| | 50:50 | 0% | 0% | 0% | 0% | 0% | 0% |
| Aromatisches Polyesterpolyol Stepan 1812 | 70:30 | 0% | 0% | 0% | 0% | 0% | 0% |
| | 50:50 | 0% | 0% | 0% | 0% | 0% | 0% |
| Aromatisches Polyesterpolyol Stepan 2412 | 70:30 | 0% | 0% | 0% | 0% | 0% | 0% |
| | 50:50 | 0% | 0% | 0% | 0% | 0% | 0% |
| Dibasicester (DBE) | 70:30 | 0% | 0% | 0% | 0% | 0% | 0% |
| | 50:50 | 0% | 0% | 0% | 0% | 0% | 0% |
| Diphenylcresylphosphat (DPCP) | 70:30 | 0% | 0% | 0% | 0% | 1% | 1% |
| | 50:50 | 0% | 0% | 0% | 0% | 1% | 1% |
| Cresol-Resol, 50 % in DEG | 90:10 | 0% | 0% | 0% | 0% | 0% | 0% |
| | 75:25 | 0% | 0% | 0% | 0% | 0% | 0% |
| | 50:50 | 0% | 0% | 0% | 0% | 0% | 0% |
| Cresol-Resol, 50 % in DEG/TEP 1:1 | 75:25 | 0% | 0% | 0% | 0% | 0% | 0% |
| | 50:50 | 0% | 0% | 0% | 0% | 0% | 0% |
| Phenol-Resol, 50 % in DEG/TEP 1:1 | 90:10 | 0% | 0% | 0% | 0% | 0% | 0% |
| | 75:25 | 0% | 0% | 0% | 0% | 0% | 0% |
| | 50:50 | 0% | 0% | 0% | 0% | 0% | 0% |

Durch den hohen aromatischen Anteil des erfindungsgemäß hergestellten alkoxylierten Produktes und gegebenenfalls zusätzlich noch durch die Verwendung von alkoxyltertem Resorcin, was den aromatischen Anteil weiter erhöht, konnte eine gute Flammbeständigkeit im Polyurethan-Endprodukt erzielt werden, wobei auch die Kompatibilität zum MDI bzw. zu den Treibmitteln weiter erhöht werden konnte. Auf den zusätzlichen Einsatz von halogenierten Flammschutzmitteln konnte demnach verzichtet werden.

## Patentansprüche

1. Verfahren zur Herstellung eines alkoxylierten Produktes durch Umsetzung von Bisphenol F mit Propylencarbonat und/oder Propylenoxid als Propoxylierungsmittel und Ethylencarbonat und/oder Ethylenoxid als Ethoxylierungsmittel, **dadurch gekennzeichnet, dass** das Propoxylierungsmittel und das Ethoxylierungsmittel in einem molaren Verhältnis von 70:30 bis 45:55 verwendet wird.

2. Verfahren nach Anspruch 1, **dadurch gekennzeichnet, dass** bezogen auf ein Mol Bisphenol F 1,6 mol bis 0,9 mol Propoxylierungsmittel und 1,6 mol bis 0,9 mol Ethoxylierungsmittel verwendet werden.

3. Verfahren nach Anspruch 2, **dadurch gekennzeichnet, dass** bezogen auf ein Mol Bisphenol F 1,3 mol bis 1,0 mol Propoxylierungsmittel und 1,3 mol bis 1,0 mol Ethoxylierungsmittel verwendet werden.

4. Verfahren nach Anspruch 3, **dadurch gekennzeichnet, dass** das molare Verhältnis zwischen Bisphenol F: Propoxylierungsmittel: Ethoxylierungsmittel im Wesentlichen gleich 1: 1 : 1 ist.

5. Verfahren nach zumindest einem der vorhergehenden Ansprüche, **dadurch gekennzeichnet, dass** als Propoxylierungsmittel Propylencarbonat verwendet wird.

6. Verfahren nach zumindest einem der vorhergehenden Ansprüche, **dadurch gekennzeichnet, dass** als Ethoxylierungsmittel Ethylencarbonat verwendet wird.

7. Verfahren nach zumindest einem der vorhergehenden Ansprüche, **dadurch gekennzeichnet, dass** die Umsetzung im alkalischen Medium bei Temperaturen von 120 °C bis 200 °C erfolgt.

8. Verfahren nach zumindest einem der vorhergehenden Ansprüche, **dadurch gekennzeichnet, dass** die Zugabe von Propylencarbonat und Ethylencarbonat zeitlich versetzt erfolgt.

9. Zusammensetzung ein alkoxyliertes Produkt hergestellt nach zumindest einem der vorhergehenden Ansprüche enthaltend.

10. Zusammensetzung nach Anspruch 9, **dadurch gekennzeichnet, dass** sie als weitere Komponente Organophosphate wie Triethylphosphat und Diphenylkresylphosphat, 1,4-Butandiol, alkoxyliertes Resorcin wie propoxyliertes Resorcin, Polyetherpolyole wie ethoxylierter Zucker, aromatische Polyesterpolyole, modifizierte oder unmodifizierte phenolische Resole, allein oder Mischungen hiervon enthält.

11. Zusammensetzung nach Anspruch 10, **dadurch gekennzeichnet, dass** das alkoxylierte Produkt im Gewichtsverhältnis zur weiteren Komponente 90: 10 bis 10:90 vorhanden ist.

12. Verwendung der Zusammensetzung nach zu zumindest einem der vorhergehenden Ansprüche 9 bis 11 zur Herstellung von Polyurethanen oder Polyisocyanuraten.

13. Verwendung nach Anspruch 12 zur Herstellung von Polyurethanen in Form von Präpolymeren, Schäumen, Isolationsmaterialien, Beschichtungen, Lacken, Elastomeren, Klebstoffen, Dichtmitteln und/oder Kompositwerkstoffen.
